# NEUE EUROPÄISCHE PATENTSCHRIFT

(11) **EP 2 152 341 B2**
(45) Veröffentlichungstag und Bekanntmachung des Hinweises auf die Entscheidung über den Einspruch: **02.04.2025**
(45) Hinweis auf die Patenterteilung: 11.12.2019
(21) Anmeldenummer: 08759561.7
(22) Anmeldetag: 13.05.2008
(51) Int. Cl.: A61M 11/00, A61M 15/00, A61L 2/02, B05B 11/00

(54) **ZERSTÄUBER IN FORM EINES INHALATORS ZUR MEDIZINISCHEN AEROSOL-THERAPIE**
ATOMIZER IN THE FORM OF AN INHALER FOR MEDICAL AEROSOL THERAPY
PULVÉRISATEUR SOUS FORME D'UN INHALATEUR POUR AÉROSOLTHÉRAPIE

(30) Priorität: 15.05.2007 DE 102007023012
(43) Veröffentlichungstag der Anmeldung: 17.02.2010
(73) Patentinhaber: Boehringer Ingelheim Pharma GmbH & Co. KG, 55216 Ingelheim am Rhein (DE)
(72) Erfinder: BOECK, Georg, 55216 Ingelheim am Rhein (DE); KADEL, Klaus, 55216 Ingelheim am Rhein (DE); MOSER, Achim, 55216 Ingelheim (DE)
(74) Vertreter: Von Rohr Patentanwälte Partnerschaft mbB
(86) Internationale Anmeldenummer: PCT/EP2008/055863
(87) Internationale Veröffentlichungsnummer: WO 2008/138936

(56) Entgegenhaltungen:
- EP-A1- 1 380 351
- EP-A1- 1 380 351
- EP-A2- 1 092 447
- EP-A2- 1 092 447
- EP-A2- 1 092 447
- WO-A1-03/002045
- WO-A1-03/002045
- WO-A1-03/002265
- WO-A1-03/002265
- WO-A1-2004/022244
- WO-A1-2004/022244
- WO-A1-2004/022244
- WO-A1-91/14468
- WO-A1-93/24164
- WO-A1-93/24164
- WO-A1-93/24164
- WO-A1-97/36690
- WO-A1-97/36690
- WO-A2-2005/061345
- WO-A2-2005/061345
- WO-A2-2006/042297
- WO-A2-2006/042297
- WO-A2-2006/042297
- WO-A2-2006/125577
- WO-A2-2006/125577
- WO-A2-2006/125577
- US-A1- 2003 187 387
- US-A1- 2003 187 387
- US-A1- 2003 187 387

## Beschreibung

Die vorliegende Erfindung betrifft einen Zerstäuber gemäß dem Oberbegriff des Anspruchs 1.

Ausgangspunkt der vorliegenden Erfindung ist ein unter der Marke RESPIMAT^{®} angebotener Zerstäuber in Form eines Inhalators, wie im Grundprinzip in der WO 91/14468 A1 und in konkreter Ausgestaltung in der WO 97/12687 A1 (Fig. 6a, 6b) sowie in Fig. 1 und 2 der anliegenden Zeichnung dargestellt. Der Zerstäuber weist als Reservoir für ein zu zerstäubendes Fluid einen einsetzbaren, starren Behälter mit einem Innenbeutel mit dem Fluid und einen Druckerzeuger mit einer Antriebsfeder zur Förderung und Zerstäubung des Fluids auf.

Problematisch ist insbesondere bei mehrfacher Verwendung des Zerstäubers, daß das Fluid mit der Zeit verkeimen kann. Bisher werden zur Verhinderung der Verkeimung üblicherweise Konservierungsmittel eingesetzt. Aufgrund zunehmender Allergien, Unverträglichkeiten, Inkompatibilitäten mit Wirk- oder Hilfsstoffen oder mit Zerstäuberbauteilen oder dergleichen ist es jedoch wünschenswert, keine Konservierungsmittel mehr zu verwenden.

Die WO 03/002265 A1 betrifft einen treibgasfreien Vernebler für ophthalmologische Applikation von konservierungsmittelfreien Flüssigkeiten. Es wird ein Vernebler geschildert, bei denen die Applikationslösung durch einen Pumpaufsatz mit Augenadapter aus einer Vorratsflasche ausgebracht wird. Der Pumpaufsatz weist eine in Ruheposition mit einem Kugelventil geschlossene Öffnung, durch die Flüssigkeit aus der Vorratsflasche in eine Drucckammer gepumpt werden kann, und ein Steigrohr auf, das von der Drucckammer zur Düse führt. Im Steigrohr befindet sich ein Druckkontrollventil. Die Druckkammer wird gebildet durch einen Druckzylinder und einen Kolben mit axialem Pumpkanal, wobei der Kolben in Ruheposition durch eine Feder an einem Anschlag gehalten wird. Zum Druckausgleich in den Vorratsbehälter nach dem Ausbringen von Flüssigkeit sind Stellen vorgesehen, an denen Außenluft in den Vorratsbehälter eindringen kann. Entlang des Luftwegs sind Mittel zur Sterilisierung der eintretenden Luft vorgesehen wie beispielsweise Sterilitätsfilter, nur für Luftdurchlässige Membranen oder oligodynamisch wirksame Substanzen.

Die WO97/36690 A1 betrifft ein antibakterielles Gerät zum Versprühen von Flüssigkeit aus einem Behälter, wobei auf dem Behälter eine Pumpe mit einer Düse angeordnet ist. Darüber ist eine Kappe mit einem inneren Kanal angeordnet, der sich zwischen der Düse und einem Hohlraum mit Sprühnebelauslass erstreckt. Im Hohlraum befindet sich ein Ventil. Außen an der Kappe befindet sich eine Schulter, die zum Betrieb der Pumpe heruntergedrückt wird.

Die WO 03/002045 A1 betrifft einen treibgasfreien Vernebler zur Applikation von Flüssigkeiten auf die Augenhornhaut oder das Augenbindegewebe.

Der vorliegenden Erfindung liegt die Aufgabe zugrunde, einen Zerstäuber anzugeben, wobei der Einsatz von Konservierungsmitteln und/oder eine Verkeimung des zu zerstäubenden Fluids vermieden oder zumindest verringert werden kann.

Die obige Aufgabe wird durch einen Zerstäuber gemäß Anspruch 1 gelöst. Vorteilhafte Weiterbildungen sind Gegenstand der Unteransprüche.

Weitere Vorteile, Merkmale, Eigenschaften und Aspekte der vorliegenden Erfindung ergeben sich aus den Ansprüchen und der folgenden Beschreibung bevorzugter Ausführungsformen anhand der Zeichnung. Es zeigt:
- Fig. 1: einen schematischen Schnitt eines bekannten Zerstäubers im ungespannten Zustand;
- Fig. 2: einen schematischen, um 90° gegenüber Fig. 1 gedrehten Schnitt des bekannten Zerstäubers im gespannten Zustand;
- Fig. 3: einen schematischen Schnitt eines vorschlagsgemäßen Zerstäubers gemäß einer ersten Ausführungsform in einem Auslieferungszustand mit eingebautem, verschlossenem Behälter;
- Fig. 4: einen schematischen Schnitt des Zerstäubers gemäß Fig. 3 im aktivierten Zustand bzw. mit geöffnetem Behälter;
- Fig. 5: einen schematischen, ausschnittsweisen Schnitt des Zerstäubers gemäß Fig. 3;
- Fig. 6: einen zu Fig. 5 korrespondierenden Schnitt einer zweiten Ausführungsform des Zerstäubers;
- Fig. 7: einen zum Fig. 5 korrespondierenden Schnitt einer dritten Ausführungsform des Zerstäubers;
- Fig. 8: eine schematische Draufsicht eines vorschlagsgemäßen Filters im geöffneten Zustand;
- Fig. 9: eine ausschnittsweise Vergrößerung von Fig. 8;
- Fig. 10: einen Schnitt des Filters entlang der Linie X-X von Fig. 8 des Filters im geschlossenen Zustand;
- Fig. 11: eine ausschnittsweise Vergrößerung von Fig. 10; und
- Fig. 12: eine perspektivische Ansicht eines Ausschnitts des offenen Filters gemäß Fig. 8.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

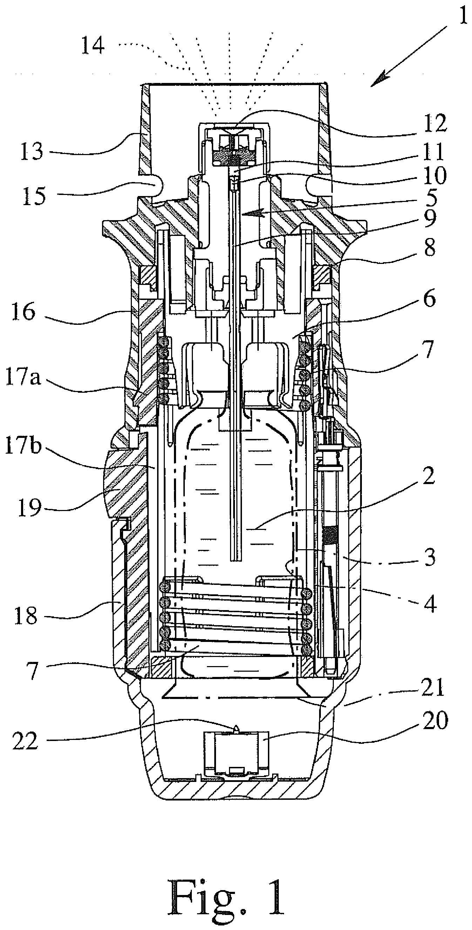

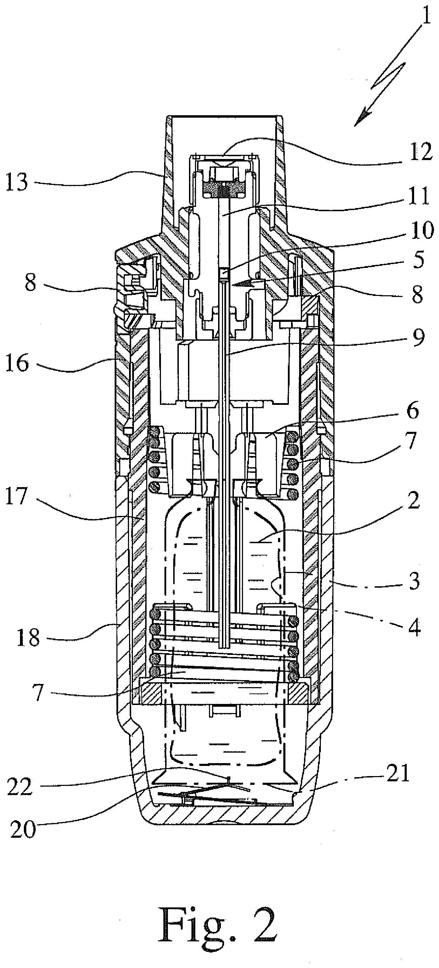

Fig. 1 und 2 zeigen einen bekannten Zerstäuber 1 zur Zerstäubung eines Fluids 2, insbesondere eines hochwirksamen Arzneimittels oder dgl., in einer schematischen Darstellung im ungespannten Zustand (Fig. 1) und gespannten Zustand (Fig. 2). Der Zerstäuber 1 ist insbesondere als tragbarer Inhalator ausgebildet und arbeitet vorzugsweise ohne Treibgas.

Bei Zerstäubung des Fluids 2, vorzugsweise einer Flüssigkeit, insbesondere eines Arzneimittels, wird ein vorzugsweise lungengängiges Aerosol 14 (Fig. 1) gebildet, das von einem nicht dargestellten Benutzer bzw. Patienten eingeatmet bzw. inhaliert werden kann. Üblicherweise erfolgt das Inhalieren wenigstens einmal täglich, insbesondere mehrmals täglich, vorzugsweise in vorbestimmten Zeitabständen, insbesondere in Abhängigkeit von der Erkrankung des Patienten.

Der bekannte Zerstäuber 1 weist einen vorzugsweise einsetzbaren und insbesondere wechselbaren Behälter 3 mit dem Fluid 2 auf. Der Behälter 3 bildet also ein Reservoir für das zu zerstäubende Fluid 2. Vorzugsweise enthält der Behälter 3 eine ausreichende Menge an Fluid 2 bzw. Wirkstoff für mehrere Dosen des Fluids 2, um beispielsweise bis zu 200 Dosiereinheiten (Dosen) zur Verfügung stellen zu können, also beispielsweise bis zu 200 Zerstäubungen oder Anwendungen zu ermöglichen. Ein typischer Behälter 3, wie in der WO 96/06011 A1 offenbart, nimmt ein Volumen von ca. 2 bis 10 ml auf.

Der Behälter 3 ist vorzugsweise im wesentlichen zylindrisch bzw. kartuschenartig ausgebildet und von unten, nach Öffnen des Zerstäubers 1, in diesen einsetzbar und ggf. wechselbar. Er ist vorzugsweise starr ausgebildet, insbesondere wobei das Fluid 2 in einem kollabierbaren Beutel 4 im Behälter 3 aufgenommen ist.

Der Zerstäuber 1 weist ferner eine Fördereinrichtung, insbesondere einen Druckerzeuger 5, zur Förderung und Zerstäubung des Fluids 2, insbesondere jeweils in einer vorbestimmten, ggf. einstellbaren Dosiermenge auf.

Der Zerstäuber 1 bzw. Druckerzeuger 5 weist insbesondere eine Halterung 6 für den Behälter 3, eine zugeordnete, nur teilweise dargestellte Antriebsfeder 7 vorzugsweise mit einem zur Entsperrung manuell betätigbaren Sperrelement 8, ein vorzugsweise als Kapillare ausgebildetes Förderrohr 9 mit einem optionalen Ventil, insbesondere Rückschlagventil 10, eine Druckkammer 11 und/oder eine Austragsdüse 12 insbesondere im Bereich eines Mundstücks 13 auf.

Der Behälter 3 wird über die Halterung 6, insbesondere klemmend oder rastend, so in dem Zerstäuber 1 fixiert, daß das Förderrohr 9 in den Behälter 3 eintaucht. Die Halterung 6 kann dabei derart ausgebildet sein, daß der Behälter 3 ausgetauscht werden kann.

Beim axialen Spannen der Antriebsfeder 7 wird die Halterung 6 mit dem Behälter 3 und dem Förderrohr 9 bei den Darstellungen nach unten bewegt und Fluid 2 aus dem Behälter 3 in die Druckkammer 11 des Druckerzeugers 5 über das Rückschlagventil 10 gesaugt.

Beim anschließenden Entspannen nach Betätigung des Sperrelements 8 wird das Fluid 2 in der Druckkammer 11 unter Druck gesetzt, indem das Förderrohr 9 bei nun geschlossenem Rückschlagventil 10 durch Entspannen der Antriebsfeder 7 wieder nach oben bewegt wird und nun als Druckstempel wirkt. Dieser Druck treibt das Fluid 2 durch die Austragsdüse 12 aus, wobei es in das vorzugsweise lungengängige Aerosol 14 zerstäubt wird, wie in Fig. 1 angedeutet.

Der nicht dargestellte Benutzer bzw. Patient kann das Aerosol 14 inhalieren, wobei vorzugsweise Zuluft über mindestens eine Zuluftöffnung 15 in das Mundstück 13 saugbar ist.

Der Zerstäuber 1 weist insbesondere ein Gehäuseoberteil 16 und ein demgegenüber drehbares Innenteil 17 (Fig. 2) mit einem oberen Teil 17a und einem unteren Teil 17b (Fig. 1) auf, wobei an dem Innenteil 17 ein insbesondere manuell betätigbares Gehäuseteil 18 vorzugsweise mittels eines Halteelementes 19 lösbar befestigt, insbesondere aufgesteckt, ist. Zum Einsetzen und/oder Auswechseln des Behälters 3 ist das Gehäuseteil 18 vom Zerstäuber 1 lösbar. Das Gehäuseteil 18 bildet vorzugsweise ein kappenartiges Gehäuseunterteil und/oder um- bzw. übergreift einen unteren freien Endbereich des Behälters 3.

Das Gehäuseteil 18 kann gegen das Gehäuseoberteil 16 gedreht werden, wobei es den in der Darstellung unteren Teil 17b des Innenteils 17 mitnimmt. Dadurch wird die Antriebsfeder 7 über ein nicht dargestelltes, auf die Halterung 6 wirkendes Getriebe in axialer Richtung gespannt. Mit dem Spannen wird der Behälter 3 axial nach unten bzw. mit seinem Endbereich (weiter) in das Gehäuseteil 18 bzw. zu dessen stirnseitigem Ende hin bewegt, bis der Behälter 3 eine in Fig. 2 angedeutete Endlage einnimmt. In diesem Zustand ist die Antriebsfeder 7 gespannt.

Beim erstmaligen Spannen erfolgt vorzugsweise ein bodenseitiges Anstechen bzw. Öffnen des Behälters 3. Insbesondere kommt eine axial wirkende, im Gehäuseteil 18 angeordnete Feder 20 am Behälterboden 21 zur Anlage, die mit einem Anstechelement 22 den Behälter 3 bzw. eine bodenseitige Versiegelung bei der erstmaligen Anlage zur Belüftung ansticht.

Während des Zerstäubungsvorgangs wird der Behälter 3 von der Antriebsfeder 7 wieder in seine Ausgangslage zurückbewegt.

Der Behälter 3 führt also eine Hubbewegung während des Spannvorgangs und während des Zerstäubungsvorgangs aus.

Es wird vorsorglich auf den kompletten Offenbarungsgehalt sowohl der WO 91/14468 A1 als auch der WO 97/12687 A1 verwiesen. Generell bezieht sich die dortige Offenbarung bevorzugt auf einen Zerstäuber mit einem Federdruck von 5 bis 60 MPa, bevorzugt 10 bis 50 MPa auf das Fluid, mit pro Hub ausgebrachtem Fluid-Volumen von 10 bis 50 µl, bevorzugt 10 bis 20 µl, ganz bevorzugt etwa 15 µl. Dabei wird das Fluid in ein Aerosol überführt, dessen Tröpfchen einen aerodynamischen Durchmesser von bis zu 20 µm, bevorzugt 3 bis 10 µm, haben. Ferner bezieht sich die dortige Offenbarung bevorzugt auf einen Zerstäuber mit zylinderähnlicher Form und einer Größe von etwa 9 cm bis etwa 15 cm in der Länge und etwa 2 cm bis etwa 5 cm in der Breite sowie mit einer Düsen-Strahlfächerung von 20° bis 160°, bevorzugt von 80° bis 100°. Derartige Werte gelten auch für den Zerstäuber nach der Lehre der vorliegenden Erfindung als besonders bevorzugte Werte.

Nachfolgend werden der Aufbau und die Funktionsweise von mehreren Ausführungsformen des vorschlagsgemäßen Zerstäubers 1 näher erläutert, wobei auf die weiteren, insbesondere nur schematischen und nicht maßstabsgerechten Fig. Bezug genommen wird und nur wesentliche Unterschiede gegenüber dem Zerstäuber 1 gemäß Fig. 1 und 2 herausgestellt werden. Die Ausführungen zu Fig. 1 und 2 gelten also entsprechend bzw. ergänzend, wobei auch beliebige Kombinationen von Merkmalen des Zerstäubers 1 gemäß Fig. 1 und 2 und der Zerstäuber 1 gemäß den nachfolgend beschriebenen Ausfiihrungsformen oder untereinander möglich sind.

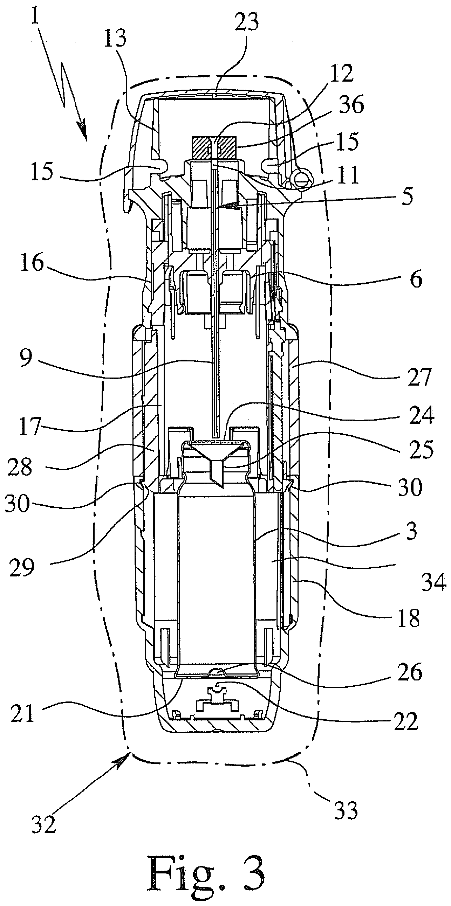

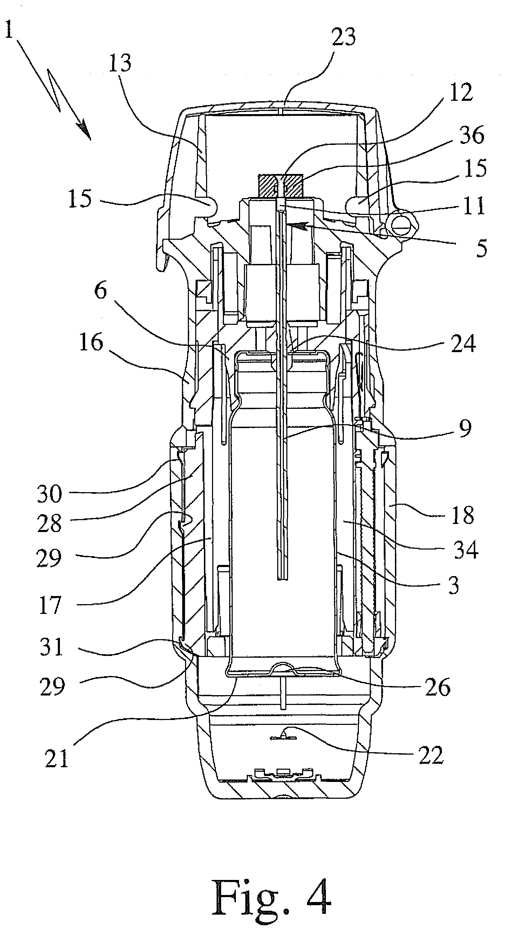

Fig. 3 und 4 zeigen in schematischen Schnitten den vorschlagsgemäßen Zerstäuber 1 gemäß einer ersten Ausführungsform. Fig. 3 zeigt den Auslieferungszustand mit verschlossenem und insbesondere versiegeltem Behälter 3. Fig. 4 zeigt den aktivierten Zustand, also mit bereits geöffnetem Behälter 3.

Fig. 3 und 4 zeigen den Zerstäuber 1 mit einer insbesondere kappenartigen, optionalen Abdeckung 23, die das Mundstück 13 und insbesondere auch die Zuluftöffnungen 15 bei Nichtgebrauch des Zerstäubers 1 abdeckt bzw. verschließt. Die Abdeckung 23 ist zum Gebrauch des Zerstäubers 1 beispielsweise abziehbar, aufklappbar oder in sonstiger Weise entfernbar oder öffenbar.

Vorzugsweise ist der (noch) verschlossene Behälter 3 bereits im Auslieferungszustand 1 im Zerstäuber 1 angeordnet, wie in Fig. 3 dargestellt. Im verschlossenen Zustand ist bzw. sind bei den dargestellten Ausführungsformen insbesondere eine kopfseitige, äußere Versiegelung 24 des Behälters 3 und/oder ein im Inneren des Behälters 3 angeordnetes Septum 25, eine Membran, ein Kunststoffverschluß oder dergleichen noch nicht geöffnet. Des weiteren ist im verschlossenen Zustand bei der dargestellten Ausführungsform eine vorzugsweise bodenseitige Belüftungsöffnung 26 des Behälters 3, die mittels des Anstechelements 22 öffenbar ist, geschlossen, also noch nicht angestochen. Es ist anzumerken, daß der Behälter 3 abhängig von der jeweiligen Konstruktion auch weniger und/oder andere Öffnungsmöglichkeiten aufweisen kann.

Der Zerstäuber 1 ist vorzugsweise derart ausgebildet, daß der Behälter 3 vor oder bei der erstmaligen Benutzung des Zerstäubers 1 innerhalb des Zerstäubers 1 geöffnet wird bzw. werden kann. Ein Öffnen des Behälters 3 liegt insbesondere bereits dann vor, wenn die Versiegelung 24 und das Septum 25 oder dergleichen geöffnet sind. Dies wird hier auch kurz als aktivierter Zustand bezeichnet. Das Anstechen bzw. Öffnen der Belüftungsöffnung 26 kann separat, insbesondere erst später beim (erstmaligen) Spannen des Zerstäubers 1 erfolgen.

Das Öffnen des Behälters 3 erfolgt vorschlagsgemäß insbesondere durch ein Förderelement, insbesondere das Förderrohr 9 oder dergleichen, vorzugsweise durch Anstechen des Behälters 3 oder Einführen in den Behälter 3. Durch entsprechende Relativbewegung, insbesondere in Längsrichtung bzw. Hubrichtung des Behälters 3 relativ zum Förderrohr 9, durchstößt das Forderrohr 9 die Versiegelung 24 und wird durch das Septum 25 hindurch in das Innere des Behälters 3, insbesondere in den Beutel 4, eingeführt, wodurch der Behälter 3 geöffnet, nämlich eine Fluidverbindung zum Austritt des Fluids 2 aus dem Behälter 3 gebildet wird. Der Behälter 3 wird also insbesondere kopfseitig geöffnet.

Während der normalen Spann- und Zerstäubungshübe wird dann der Behälter 3 vorzugsweise zusammen mit dem Förderelement bzw. Förderrohr 9 mittels der Halterung 6 bewegt, wobei die hergestellte Fluidverbindung erhalten, der Behälter 3 vorzugsweise also immer geöffnet bleibt.

Das bereits erwähnte, vorzugsweise bodenseitige Belüften durch Öffnen der Belüftungsöffnung 26, kann je nach Ausführungsform oder Bedarf vor oder bei oder nach dem genannten, insbesondere kopfseitigen Öffnen des Behälters 3 erfolgen.

Bei der ersten Ausführungsform ist der Behälter 3 vorinstalliert und das Gehäuseteil 18 im Auslieferungszustand axial nicht vollständig aufgeschoben. Vielmehr ist insbesondere zwischen dem Gehäuseteil 18 und dem Gehäuseoberteil 16 beispielsweise ein Sicherungsteil 27 angeordnet, so daß das Gehäuseteil bzw. Unterteil 18 ausreichend weit vom Gehäuseoberteil 16 abgerückt ist, um den (noch) verschlossenen Behälter 3 axial abgerückt vom Förderrohr 9 halten zu können.

Das Gehäuseteil 18 ist in dem nicht aktivierten, abgerückten Zustand vorzugsweise durch mindestens einen am Gehäuseoberteil 16 oder Innenteil 17 angeordneten Rastarm 28 oder dergleichen unverlierbar und insbesondere unlösbar gehalten. Vorzugsweise greift der Rastarm 28 mit einer Rastnase 29 in eine Rastausnehmung 30 im Gehäuseteil 18 ein und sichert dadurch das Gehäuseteil 18 formschlüssig gegen ein vollständiges axiales Abziehen. Jedoch sind hier auch andere konstruktive Lösungen möglich.

Insbesondere ist das Gehäuseteil bzw. Unterteil 18 des Zerstäubers 1 nach dem erstmaligen (teilweisen) axialen Aufschieben nicht mehr vom Zerstäuber 1 lösbar, der Zerstäuber 1 also nicht mehr öffenbar, so daß der Behälter 3 nicht auswechselbar, insbesondere nicht wieder entnehmbar ist.

Das Sicherungsteil 27 ist beispielsweise im wesentlichen hohlzylindrisch ausgebildet und axial zwischen dem Gehäuseteil 18 und dem Gehäuseoberteil 16 angeordnet. Um den Zerstäuber 1 zu aktivieren, also das Gehäuseteil 18 vollständig axial aufschieben und dadurch den Behälter 3 öffnen zu können, muß zunächst das Sicherungsteil 27 entfernt oder überwunden werden. Insbesondere ist das Sicherungsteil 27 in der Art einer Banderole oder dergleichen, beispielsweise aus Kunststoff, ausgebildet und kann manuell geöffnet, entfernt, überwunden, zerbrochen, zerschnitten oder zerstört werden. Das Sicherungsteil 27 kann alternativ oder gleichzeitig einen Originalitätsverschluß bilden oder darstellen. Jedoch sind auch andere Ausführungen des Sicherungsteils 27 möglich, beispielsweise in Form einer Sicherungslasche oder dergleichen. Zur Sicherung kann anstelle des Sicherungsteils 27 auch jede sonstige geeignete Sicherung eingesetzt werden. Beispielsweise ist es möglich, daß das Gehäuseteil 18 zunächst etwas gedreht werden muß, um dann axial (vollständig) aufgeschoben werden zu können. Mögliche Realisierungen der Sicherung werden insbesondere in der WO 2006/125577 A1 offenbart.

Nach oder bei Entfernen oder Überwinden des Sicherungsteils 27 bzw. einer sonstigen Sicherung kann ein nicht dargestellter Benutzer das Gehäuseteil 18 in axialer Richtung vollständig aufschieben und dadurch den aktivierten Zustand des Zerstäubers 1 herbeiführen, nämlich den Behälter 3 durch Einführen des Förderelements bzw. Förderrohrs 9 öffnen. Fig. 4 zeigt diesen aktivierten Zustand mit vollständig aufgeschobenem Gehäuseteil 18. In diesem aufgeschobenen Zustand wird das Gehäuseteil 18 vorzugsweise wieder formschlüssig gesichert bzw. gehalten, insbesondere durch Eingriff des Rastarms 28 bzw. der Rastnase 29 in eine entsprechende, weitere Rastausnehmung 31 oder durch eine sonstige mechanische Sicherung.

Fig. 4 zeigt den Zerstäuber 1 bzw. den Behälter 3 im aktivierten Zustand, der Behälter 3 ist bereits geöffnet und das Gehäuseteil 18 ist vollständig axial aufgeschoben. Um die Halterung 6 mit dem Behälter 3 kopfseitig in Eingriff zu bringen und darüber dann den Behälter 3 für die Spann- und Druckhübe bewegen zu können, kann ein erstmaliges Spannen des Zerstäubers 1 erforderlich sein. Während dieses Spannvorgangs wird die Halterung 6 zusammen mit dem Förderrohr 9 axial zum bzw. in das Gehäuseteil 18 bewegt, wodurch die Halterung 6 in Eingriff mit dem Behälter 3 gebracht und vorzugsweise auch der Behälter 3 gegen das Anstechelement 22 im Bereich des Bodens des Gehäuseteils 18 gedrückt und dadurch die Belüftungsöfnung 26 angestochen bzw. geöffnet wird. Fig. 4 zeigt den Zerstäuber 1 im entspannten Zustand, also insbesondere nach der ersten Zerstäubung. Die Halterung 6 steht mit dem Behälter 3 in Eingriff, und das Förderrohr 9 ist vollständig in den Behälter 3 eingeführt.

In dem in Fig. 3 dargestellten Auslieferungszustand, also mit (noch) verschlossenem Behälter 3, kann der Zerstäuber 1 gelagert werden. Insbesondere sorgt die geschlossene Versiegelung 24 dafür, das ein im Fluid 2 bedarfsweise enthaltenes Lösungsmittel nicht oder allenfalls nur in sehr geringem Maß entweichen kann.

Um ein unerwünschtes Öffnen des Behälters 3, insbesondere der Versiegelung 24 oder der Belüftungsöffnung 26, im Auslieferungszustand des Zerstäubers 1 zu verhindern, weist der Zerstäuber 1 vorzugsweise eine nicht dargestellte Transportsicherung auf. Die Transportsicherung verhindert beispielsweise durch Reib-, Kraft- oder Formschluß, daß sich der Behälter 3 in unerwünschter Weise axial im Zerstäuber 1 - beispielsweise während des Transports, bei einem unbeabsichtigten Herunterfallen des Zerstäubers 1 oder dergleichen - bewegen und dadurch ungewollt öffnen kann.

Möglichkeiten der Transportsicherung des Behälters 3 werden insbesondere in der WO 2006/125577 A1 offenbart.

Es ist anzumerken, daß das Öffnen des Behälters 3 vorzugsweise ausschließlich durch mechanische Einwirkung und/oder manuelle Betätigung erfolgt. Jedoch ist zusätzlich oder alternativ auch ein Öffnen auf andere Weise, beispielsweise chemisch, elektrisch, magnetisch, pneumatisch, hydraulisch oder dergleichen, möglich,

Der vorschlagsgemäße Zerstäuber 1 ist nach vollständigem axialem Aufschieben des Behälters 3 bzw. Gehäuseteils 18 aktiviert und entsprechend dem in Fig. 1 und 2 dargestellten Zerstäuber 1 benutzbar.

Im Gegensatz zu Standgeräten oder dergleichen ist der vorschlagsgemäße Zerstäuber 1 vorzugsweise transportabel ausgebildet, insbesondere handelt es sich um ein mobiles Handgerät.

Der vorschlagsgemäße Zerstäuber arbeitet insbesondere rein mechanisch. Jedoch kann der Zerstäuber 1 grundsätzlich auch auf jede sonstige Art und Weise arbeiten. Insbesondere ist der Begriff "Fördereinrichtung" bzw. "Druckerzeuger" sehr allgemein zu verstehen. Beispielsweise kann der zur Ausgabe und Zerstäubung erforderliche Druck auch durch Treibgas, eine Pumpe oder auf jede sonstige geeignete Art und Weise erzeugt werden.

Der vorschlagsgemäße Zerstäuber 1 ist insbesondere zur kurzzeitigen Zerstäubung des Fluids 2, beispielsweise für ein bis zwei Atemzüge, ausgebildet. Jedoch kann er auch zur längeren oder kontinuierlichen Zerstäubung ausgebildet bzw. einsetzbar sein.

Der Zerstäuber 1 weist vorzugsweise eine Schutzeinrichtung 32 zur Verhinderung oder zumindest Verminderung einer möglichen Verkeimung des Fluids 2 auf.

Bei der ersten Ausführungsform weist die Schutzeinrichtung 32 eine keimdichte, insbesondere sterile Umhüllung, vorzugsweise einen Beutel 33, zur Aufnahme des Zerstäubers 1 und vorzugsweise auch des Behälters 3 im Auslieferungszustand auf, wie in Fig. 3 dargestellt. Die Umhüllung bzw, der Beutel 33 ist vorzugsweise aus Folien- oder Plastikmaterial hergestellt und/oder zumindest teilweise transparent ausgebildet. Die Umhüllung bzw. der Beutel 33 ist vorzugsweise gasdicht ausgebildet.

Die Umhüllung bzw. der Beutel 33 umgibt den Zerstäuber 1 vorzugsweise relativ lose und/oder ist insbesondere flexibel oder elastisch, so daß der Zerstäuber bzw. Behälter 3 vorzugsweise bei noch geschlossener Umhüllung aktivierbar ist.

Nach der Aktivierung kann die Umhüllung geöffnet, insbesondere aufgerissen oder aufgeschnitten, und entfernt werden, so daß der Zerstäuber dann ganz normal einsetzbar ist. In Fig. 4 ist der Beutel 33 bereits entfernt.

Die Schutzeinrichtung 32 bzw. die Umhüllung führt insbesondere dazu, daß der Zerstäuber 1 und der Behälter 3 sehr lange keimdicht und insbesondere steril lagerbar sind.

Gemäß einer ersten Verfahrensvariante wird der bereits sterile Behälter 3 unter sterilen Bedingungen in den sterilen Zerstäuber 1 eingesetzt und der Zerstäuber 1 dann unter sterilen Bedingungen von der ebenfalls sterilen Umhüllung umschlossen bzw, eingeschlossen. Eine anschließende oder zusätzliche Sterilisierung ist dann nicht mehr erforderlich, aber möglich.

Gemäß einer zweiten Verfahrensvariante erfolgt das Sterilisieren erst nachdem der Behälter 3 in den Zerstäuber 1 eingesetzt worden ist und gegebenenfalls sogar erst nachdem der Zerstäuber 1 von der Umhüllung eingeschlossen ist. In diesem Fall muß der Zerstäuber 1, Behälter 3 und/oder die Umhüllung nicht vorab sterilisiert sein bzw. werden und/oder muß nicht unter sterilen Bedingungen gearbeitet werden.

Die Sterilisierung kann insbesondere durch Strahlung (z. B. Mikrowellen-, UV-, Röntgen- oder Gammastrahlung) und/oder Gas (z. B. Ethylenoxid) erfolgen. Gegebenenfalls kann es auch genügen, beim Verschließen die Umhüllung mit einem sterilisierenden Gas zu füllen.

Weiter kann die Schutzeinrichtung 32 alternativ oder zusätzlich einen keimdichten Aufnahmeraum 34 für den Behälter 3 im Zerstäuber 1 aufweisen. Insbesondere ist der Aufnahmeraum 34 vom Zerstäuber 1 gebildet, besonders bevorzugt durch das Gehäuseteil 18, das dann in diesem Fall keimdicht aufsetzbar ist. Jedoch sind hier auch andere konstruktive Lösungen möglich.

Das Sterilisieren erfolgt dann wie bei dem Beutel 33 vorzugsweise vor, beim und/oder nach dem Schließen.

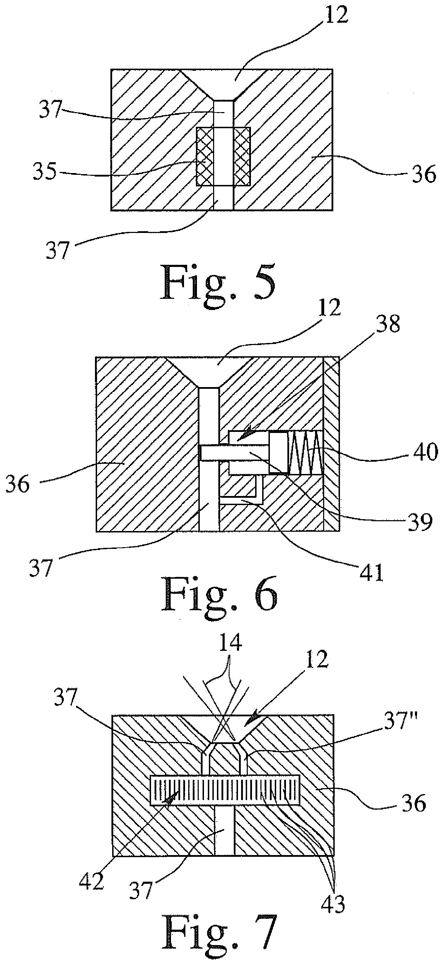

Gemäß einer weiteren Ausführungsvariante kann die Schutzeinrichtung 32 einen Bakterien oder sonstige Keime abtötenden oder antibakteriell wirkenden Kontaktabschnitt 35 für das Fluid 2 aufweisen. Der Kontaktabschnitt 35 ist in Fig. 5 gezeigt, die einen schematischen, vergrößerten Schnitt eines Teils des Zerstäubers 1, insbesondere einer Auslaß- oder Düsenbaugruppe 36 zeigt.

Der Kontaktabschnitt 35 wirkt insbesondere oligodynamisch. Vorzugsweise enthält der Kontaktabschnitt 35 Silber und/oder Kupfer, derartige Legierungen, Mischungen oder dergleichen oder ist daraus hergestellt. Alternativ oder zusätzlich kann der Kontaktabschnitt 35 auch Aktivkohle enthalten oder daraus hergestellt sein.

Der Kontaktabschnitt 35 ist vorzugsweise in einem Abschnitt des Fluidwegs, insbesondere in der Auslaß- bzw. Düsenbaugruppe 36, in einem Auslaßkanal 37 bzw. in der Fördereinrichtung oder, dem Druckerzeuger 5 oder in oder an dem Behälter 3, angeordnet oder davon gebildet.

Besonders bevorzugt ist der Kontaktabschnitt 35 in der Nähe der Austragsdüse 12 oder eines sonstiges Auslasses und/oder stromab der Druckkammer 11 angeordnet.

Der Kontaktabschnitt 35 kann bedarfsweise alternativ oder zusätzlich auch in der Druckkammer 11 angeordnet sein. Alternativ oder zusätzlich kann der Kontaktabschnitt 35 auch am Behälter 3, insbesondere an dessen Auslaß und/oder am oder im Förderrohr 9 oder dergleichen angeordnet sein.

Grundsätzlich kann der Kontaktabschnitt 35 jedoch auch an sonstigen geeigneten Stellen angeordnet sein. Bedarfsweise können auch mehrere Kontaktabschnitte 35 entlang des Fluidwegs vorgesehen sein.

Beim Darstellungsbeispiel bildet der Kontaktabschnitt 35 insbesondere einen Teil einer Wandung eines Kanals, wie des Auslaßkanals 37. Hierzu ist der Kontaktabschnitt 35 insbesondere hohlzylindrisch ausgebildet oder mit einer entsprechenden Bohrung, Durchbrechung oder dergleichen versehen. Jedoch sind auch andere konstruktive Lösungen möglich. Insbesondere ist es auch möglich, daß der Kontaktabschnitt 35 eine Flachseite oder gegenüberliegende Flachseiten eines sehr flachen Kanalabschnitts bildet, um eine möglichst große Kontaktfläche zwischen dem Kontaktabschnitt 35 einerseits und dem Fluid 2 andererseits zu erreichen.

Der Kontaktabschnitt 35 kann generell durch ein Bauteil oder einen Abschnitt eines Bauteils des Zerstäubers 1 oder eine zumindest bereichsweise Beschichtung gebildet sein.

Alternativ oder zusätzlich kann der Kontaktabschnitt 35 auch durch eine Materialbeimischung und/oder insbesondere diskrete Partikel oder sonstige, insbesondere oligodynamisch bzw. antibakteriell wirkende Partikel gebildet sein oder können derartige Partikel in ein Material (beispielsweise Metall, Verbundwerkstoff, Kunststoff oder Keramik), integriert sein, so daß die Partikel mit dem Fluid 2 wechselwirken können. Alternativ oder zusätzlich können die Partikel auch in einem Oberflächenbereich angeordnet sein bzw. eine Beschichtung bilden. Gemäß einer Ausführungsvariante ist das Forderelement bzw. Förderrohr 9 aus einem entsprechenden, antibakteriell wirkenden Material, wie einer silberhaltigen Legierung, hergestellt und/oder damit beschichtet. Alternativ oder zusätzlich können auch Kunststoffoberflächen entsprechend zur Bildung des Kontaktabschnitts 35 beschichtet sein.

Gemäß einer Ausführungsvariante ist der Kontaktabschnitt 35 an oder in der Austragsdüse 12 oder Auslaß- bzw. Düsenbaugruppe 36 gebildet.

Besonders bevorzugt ist der Kontaktabschnitt 35 in einem Resttropfenbereich auf der Außenseite der Düse 12 oder Auslaß- bzw. Düsenbaugruppe 36 gebildet oder angeordnet.

Alternativ oder zusätzlich kann der Kontaktabschnitt 35 auch in einen Filter integriert oder davon gebildet sein.

Gemäß einer besonders bevorzugten Ausführungsvariante sind alle mit dem Fluid 2 in Kontakt tretenden Metallbauteile des Zerstäubers 1 zumindest im wesentlichen aus dem gleichen Material und/oder aus Material mit zumindest im wesentlichen gleicher Elektronegativität hergestellt und/oder damit beschichtet.

Die Auslaß- bzw. Düsenbaugruppe 36 weist vorzugsweise den Auslaßkanal 37 mit der sich anschließenden Austragsdüse 12 auf. Besonders bevorzugt ist die Auslaß- bzw. Düsenbaugruppe 36 einstückig - gegebenenfalls mit Ausnahme des Kontaktabschnitts 35 - hergestellt, besonders bevorzugt aus Keramik, Sintermaterial, Silizium, Glas oder dergleichen.

Die Auslaß- bzw. Düsenbaugruppe 36 ist vorzugsweise einlaßseitig an die Druckkammer 11 fluidisch angeschlossen. Insbesondere innerhalb des Mundstücks 13 so eingebaut, daß bei der Fluidausgabe die gewünschte Zerstäubung des Fluids 2 als Aerosol 14 in und/oder aus dem Mundstück 13 erfolgt.

Alternativ oder zusätzlich kann die Abdeckung 23 auch als Schutzeinrichtung 32 ausgebildet sein. In diesem Fall ist die Abdeckung 23 vorzugsweise keimdicht schließend ausgebildet, um eine mögliche Verkeimung des Fluids 2 über die Austragsdüse 12 zu verhindern oder zumindest zu verringern. Bedarfsweise kann die Abdeckung 23 hierzu auch unmittelbar auf der Austragsdüse 12 bzw. der Auslaß- oder Düsenbaugruppe 36 zur Anlage kommen. Alternativ oder zusätzlich kann die Abdeckung 23 insbesondere in diesem Bereich oder die Auslaß- bzw. Düsenbaugruppe 36 in diesem Bereich oligodynamisch ausgebildet sein bzw. dort einen Kontaktabschnitt 35 im bereits genannten Sinne aufweisen oder bilden.

Nachfolgend wird eine zweite Ausführungsform des vorschlagsgemäßen Zerstäubers 1 anhand von Fig. 6 beschrieben, wobei insbesondere nur auf wesentliche Unterschiede gegenüber der ersten Ausführungsform eingegangen wird. Die bisherigen Ausführungen und Erläuterungen gelten daher insbesondere entsprechend oder ergänzend.

Fig. 6 zeigt in einer zu Fig. 5 korrespondierenden Darstellung einen schematischen, ebenfalls nicht maßstabsgerechten Schnitt der Auslaß- bzw. Düsenbaugruppe 36 des Zerstäubers 1 gemäß der zweiten Ausführungsform.

Bei der zweiten Ausführungsform weist die Schutzeinrichtung 32 zusätzlich oder alternativ zu den bereits beschriebenen Möglichkeiten ein sperrbares Ventil 38 zur Verhinderung oder zumindest Minimierung einer Verkeimung des Fluids 2 im Behälter 3, in dem Förderelement, wie dem Förderrohr 9, in der Druckkammer 11, in dem Auslaßkanal 37 und/oder in einem sonstigen Fluid führenden Abschnitt bzw. generell in der Fördereinrichtung bzw. dem Druckerzeuger 5 auf. Das Ventil 38 sperrt insbesondere einen zugeordneten Kanal, wie den Auslaßkanal 37, um ein Eindringen von Keimen zu verhindern oder zumindest zu minimieren.

Beim Darstellungsbeispiel ist das Ventil 38 der Auslaß- bzw. Düsenbaugruppe 36 zugeordnet, insbesondere in diese eingebaut. Jedoch sind auch andere konstruktive Lösungen möglich.

Das Ventil 38 ist vorzugsweise selbstätig schließend, insbesondere durch Federkraft, ausgebildet. Beispielsweise ist ein bewegliches Ventilelement 39 mittels einer Schließfeder 40 in die in Fig. 6 gezeigte Schließposition vorgespannt, in der es den Auslaßkanal 37 sperrt.

Das Ventil 38 ist vorzugsweise durch den anstehenden Fluiddruck öffenbar. Beim Darstellungsbeispiel ist hierzu ein Verbindungskanal 41 vorgesehen, der stromauf des Ventils 38 vom Auslaßkanal 37 abzweigt, um bei entsprechendem Fluiddruck das Ventilelement 39 gegen die Kraft der Schließfeder 40 öffnen zu können. Jedoch sind hier auch andere konstruktive Lösungen möglich.

Insbesondere kann das Ventil 38 als Einweg- oder Rückschlagventil ausgebildet sein.

Beim Darstellungsbeispiel arbeitet das Ventil 38 vorzugsweise ausschließlich mechanisch bzw. hydraulisch. Jedoch sind auch andere konstruktive Lösungen möglich. Beispielsweise kann das Ventil 38 auch elektrisch, elektromagnetisch, piezoelektrisch, pneumatisch oder auf sonstige geeignete Weise arbeiten und insbesondere geöffnet und/oder geschlossen werden.

Das Ventil 38 ist vorzugsweise zur Sperrung des Auslaßkanals 37 oder eines sonstigen Kanalabschnitts zur Führung des Fluids 2 ausgebildet. Jedoch kann es gemäß einer nicht dargestellten Ausführungsvariante auch genügen, den Fluidstrom bzw. die Fluidsäule zu unterbrechen, beispielsweise durch einen entsprechenden Kapillarstop oder dergleichen, so daß beispielsweise zwei getrennte Fluidbereiche oder -säulen gebildet werden, die nicht unmittelbar fluidisch miteinander in Kontakt stehen, wodurch die Übertragung von Keimen dazwischen entsprechend unterbunden oder zumindest erschwert wird.

Das Ventil 38 kann wie der Kontaktabschnitt 35 an verschiedenen Stellen angeordnet sein. Insbesondere ist das Ventil 38 unmittelbar benachbart oder stromauf der Austragsdüse 12 und/oder stromab der Druckkammer 11 angeordnet. Zusätzlich oder alternativ kann das Ventil 38 auch am Behälter 3, insbesondere zum Sperren seines Auslasses, angeordnet sein.

Bedarfsweise kann der Kontaktabschnitt 35 auch in das Ventil 38 integriert sein oder umgekehrt.

Der schematische, zu den Fig. 5 und 6 korrespondierende Schnitt gemäß Fig. 7 zeigt eine dritte Ausführungsform des vorschlagsgemäßen Zerstäubers 1. Dargestellt ist wiederum nur die Auslaß- bzw. Düsenbaugruppe 36. Die bisherigen Ausführungen und Erläuterungen gelten entsprechend.

Bei der dritten Ausführungsform ist als Schutzeinrichtung 32 zusätzlich oder alternativ zu den bereits beschriebenen Möglichkeiten ein Filter 42 vorgesehen, der nur so feine Durchlässe 43 aufweist, das er zumindest im wesentlichen keimdicht ist. Keime, wie Bakterien oder dergleichen, können also die Durchlässe 43 zumindest zum größten Teil nicht passieren.

Der Filter 42 ist vorzugsweise in dem Auslaßkanal 37 oder einem sonstigen Kanalabschnitt angeordnet, so daß das Fluid 2 insbesondere unmittelbar vor der Ausgabe über die Austragsdüse 12 oder einen sonstigen Auslaß zunächst zwangsweise den Filter 42 passieren muß. Der Filter 42 kann dann - entsprechend dem Kontaktabschnitt 35 oder Ventil 38 - ein unerwünschtes auslaßseitiges bzw. düsenseitiges Eindringen von Keimen verhindern oder zumindest minimieren.

Der Filter 42 ist beim Darstellungsbeispiel stromab der Druckkammer 11 bzw. Fördereinrichtung bzw. einer Pumpe oder dem Druckerzeuger 5 und/oder stromauf der Austragsdüse 12 angeordnet.

Der Zerstäuber 1 kann auch mehrere Austragsdüsen 12 aufweisen, wie in Fig. 7 beispielhaft gezeigt. Die Austragsdüsen 12 sind dann wahlweise über einen gemeinsamen Kanal oder - wie dargestellt - über separate Auslaßkanale 37' und 37" angeschlossen, hier insbesondere an den Filter 42.

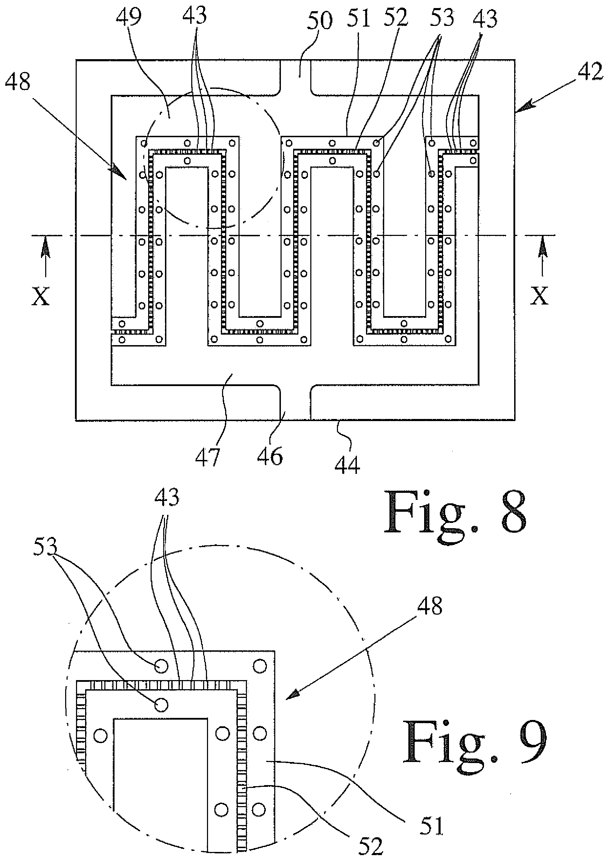

Fig. 8 bis 12 zeigen einen vorschlagsgemäßen Filter 42, der insbesondere als Schutzeinrichtung 32 bei dem vorschlagsgemäßen Zerstäuber 1 - besonders bevorzugt bei der dritten Ausführungsform, insbesondere integriert in die Auslaß- bzw. Düsenbaugruppe 36 - oder bei sonstigen Zerstäubern 1, Inhalatoren oder dergleichen einsetzbar ist. Der Filter 42 ist insbesondere zur Verhinderung oder zumindest Minimierung der möglichen Verkeimung eines vorzugsweise zu zerstäubenden Fluids 2 vorgesehen bzw. verwendbar. Das Fluid 2 kann jedoch auch auf jede sonstige geeignete Weise ausgegeben werden.

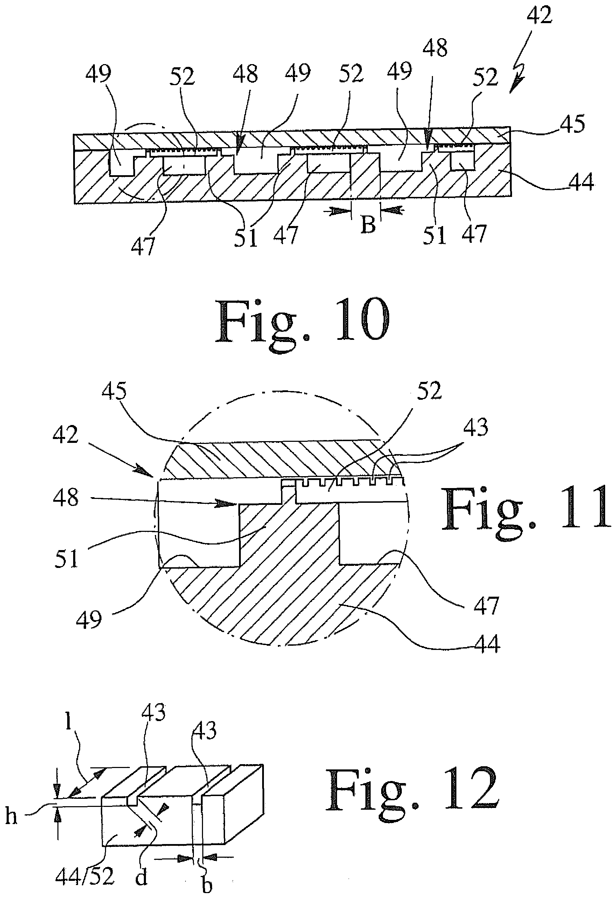

Fig. 8 zeigt den offenen Filter 42 in einer Draufsicht. Fig. 9 zeigt einen vergrößerten Ausschnitt von Fig. 8. Fig. 10 zeigt den geschlossenen Filter 42 in einem schematischen Schnitt entlang der Linie X-X von Fig. 8. Fig. 11 zeigt einen vergrößerten Ausschnitt von Fig. 10. Fig. 12 zeigt in einer perspektivischen Ansicht einen Ausschnitt des Filters 42 mit nicht abgedeckten Durchlässen 43.

Der Filter 42 weist nur so feine Durchlässe 43 auf, dass er zumindest im wesentlichen keimdicht ist. Der mittlere oder größte Durchmesser d ( Fig. 12 ) oder eine größte Querschnittserstreckung der Durchlässe 43 beträgt etwa 0,3 µm oder weniger.

Die Durchlässe 43 sind vorzugsweise im Querschnitt im wesentlichen rechteckig. Die maximale oder mittlere Breite b und/oder die maximale oder mittlere Höhe h und/oder der maximale Durchmesser d der Durchlässe 43 beträgt höchstens 0,3 µm oder weniger. Beim Darstellungsbeispiel betragen die Breiten b und die Höhe h jeweils im wesentlichen 0,2 µm.

Die Durchlässe 43 sind vorzugsweise jeweils länger als ihr maximaler Durchmesser d ausgebildet. Dies ist einer hohen Stabilität und insbesondere einer hohen Druckbeständigkeit des Filters 42 zuträglich. Insbesondere beträgt ihre Länge 1 etwa 1 µm bis 5 µm.

Der Filter 42 ist vorzugsweise aus einem Materialstück 44 und einem Abdeckelement 45 aufgebaut. Fig. 8 zeigt den Filter 42 bzw. das Materialstück 44 in Draufsicht ohne das Abdeckelement 45. Der Schnitt gemäß Fig. 10 zeigt den Filter 42 bzw. das Materialstück 44 mit dem Abdeckelement 45.

Die Durchlässe 43 sind beim Darstellungsbeispiel ausgehend von einer Flachseite des Materialstücks 44 ausgenommen, wie insbesondere in Fig. 10 angedeutet.

Vorzugsweise werden die Durchlässe 43 also zwischen dem Materialstück 44 und dem Abdeckelement 45 gebildet. Jedoch sind auch andere konstruktive Lösungen möglich.

Beim Darstellungsbeispiel wird das Materialstück 44 ausgehend von einer Flachseite ausgenommen und in gewünschter Weise strukturiert, insbesondere durch Ätzen, beispielsweise wie aus der Halbleitertechnik bekannt, durch Prägen, durch Laserbearbeitung und/oder durch jede sonstige geeignete Bearbeitungsweise. Beispielsweise können größere Bereiche auch weggefräst werden. Wesentlich ist die Bearbeitungsgenauigkeit, da sehr feine, definierte Durchlässe 43 gebildet werden.

Beim Darstellungsbeispiel liegen die Durchlässe 43 vorzugsweise im wesentlichen in einer Ebene, die parallel zur Durchströmung der Durchlässe 43 verläuft.

Die Durchlässe 43 sind vorzugsweise von einer gemeinsamen Abdeckung, nämlich dem Abdeckelement 45, seitlich begrenzt bzw. abgedeckt.

Die Durchlässe 43 sind vorzugsweise linienförmig nebeneinander angeordnet und verlaufen insbesondere mäanderförmig (Fig. 8) oder zickzackförmig oder auf jede sonstige geeignete Art und Weise.

Beim Darstellungsbeispiel weist der Filter 42 vorzugsweise einen Einlaß 46 und eine sich anschließende Verteilkammer 47 auf. Eine Filterstruktur 48 mit den Durchlässen 43 trennt die Verteilkammer 47 von einer auf der anderen Seite der Filterstruktur 48 befindlichen Sammelkammer 49, die an einen Auslaß 50 des Filters 42 angeschlossen ist.

Der Einlaß 46 ist vorzugsweise mit der Druckkammer 11 bzw. dem Auslaßkanal 37 verbunden. Der Auslaß 50 ist vorzugsweise mit dem Auslaßkanal 37 bzw. der Austragsdüse 12 verbunden. Insbesondere ist der Filter 42 derart angeschlossen, daß das in den Fig. 8 bis 12 nicht dargestellte Fluid 2 beim Austrag zwangsweise den Filter 42 passieren muß, also zwangsweise durch die Durchlässe 43 strömen muß.

Beim Darstellungsbeispiel sind die Durchlässe 43, der Einlaß 46, die Verteilkammer 47, die Sammelkammer 49 und/oder der Auslaß 50 vorzugsweise als Vertiefungen in dem Materialstück 44 gebildet und insbesondere von einem stehengebliebenen Rand des Materialstücks 44 umgeben, über den die Verbindung mit dem Abdeckelement 45 erfolgt.

Die Filterstruktur 48 verläuft insbesondere im wesentlichen mäanderförmig, zickzackförmig, linienförmig oder auch auf jede sonstige geeignete Art und Weise, besonders bevorzugt so, daß möglichst viele Durchlässe 43 gebildet werden können.

Beim Darstellungsbeispiel weist der Filter 42 beispielsweise mehr als 1.000 Durchlässe 43 bei verhältnismäßig geringem Gesamtvolumen und insbesondere sehr geringem Aufnahmevolumen an Fluid 2 (vorzugsweise etwa 1 µl oder weniger) auf.

Die Vielzahl der parallel geschalteten Durchlässe 43 führt zu einem vergleichsweise oder zumindest ausreichend geringen Strömungswiderstand.

Um eine hohe Stabilität der Filterstruktur 48 mit den Durchlässen 43 zu erreichen, weist die Filterstruktur 48 zunächst eine vorzugsweise rippenartige oder stegartige Erhöhung 51 auf, die sich vom Boden der Verteilkammer 47 bzw. Sammelkammer 49 aus in Richtung des Abdeckelements 45 erhebt. Die Erhebung 51 weist beispielsweise eine Breite B von einigen µm, beim Darstellungsbeispiel etwa 5 µm, auf. Die Erhebung 51 ist insbesondere durchgängig, fortlaufend und/oder linienartig ausgebildet und verläuft insbesondere mäanderförmig, zickzackförmig, linienförmig oder dergleichen entsprechend der Filterstruktur 48.

Auf der Erhebung 51 ist eine vorzugsweise schmälere, insbesondere steg-, rippen- oder streifenartige Erhöhung 52 angeordnet, die entlang der Erhebung 51 verläuft und beispielsweise eine auf etwa 5 bis 50 % gegenüber der Erhebung 51 reduzierte Breite aufweist. Die Höhe und Breite der Erhöhung 52 beträgt beispielsweise etwa jeweils 1 µm. Je nach gewünschter Länge der Durchlässe 43 kann die Breite auch beispielsweise bis 5 µm betragen. Entsprechendes gilt auch für Höhe zur möglichen Variation der Tiefe bzw. des Querschnitts der Durchlässe 43.

Ausgehend von der Flachseite der Erhöhung 52 sind die Durchlässe 43 durch insbesondere Rillen oder nutartige Ausnehmungen bzw. Vertiefungen in der Erhöhung 52 gebildet.

Der Mittenabstand der Durchlässe 43 beträgt beispielsweise etwa 1 µm oder der Breite bzw. Dicke der Erhöhung 52. Jedoch sind auch andere Dimensionierungen möglich.

Bei der Darstellung gemäß Fig. 8 weist die Flachseite des Materialstücks 44 und die Flachseite der Erhöhung 52 zum Betrachter. Die Durchlässe 43 sind an der Flachseite - also längsseitig - noch offen und werden vorzugsweise erst durch das Abdeckelement 45 oder eine sonstige Abdeckung abgedeckt und seitlich geschlossen. Dies erleichtert die Herstellung mit hoher Genauigkeit bzw. geringen Toleranzen - insbesondere durch Ätzen - wesentlich. Jedoch sind auch andere konstruktive Lösungen möglich.

Um die besondere Verbindung mit dem Abdeckelement 45 noch zu verbessern und/oder die Stabilität der Filterstruktur 48 im Filter 42 weiter zu erhöhen, insbesondere im Hinblick auf eine besonders hohe Druckstabilität und sonstige Festigkeit des Filters 42, können optional Stützstrukturen 53 vorgesehen sein, wie in Fig. 8 angedeutet. Die Stützstrukturen 53 erstrecken sich von der Erhebung 51 aus neben der Erhöhung 52 zum Abdeckelement 45, mit dem diese vorzugsweise auch verbindbar sind. In Fig. 10 sind die optionalen Stützstrukturen 53 aus Vereinfachungsgründen nicht dargestellt.

Als Ausgangsmaterial wird vorzugsweise Vollmaterial bzw. Plattenmaterial für das Materialstück 44 eingesetzt.

Das Materialstück 44 besteht vorzugsweise aus Silizium oder einem sonstigen geeigneten Werkstoff, beispielsweise Sintermaterial, Keramik, Glas oder dergleichen. Das Abdeckelement 45 besteht insbesondere aus demselben Werkstoff oder einem sonstigen geeigneten Werkstoff, vorzugsweise Glas. Das Verbinden des Materialstücks 44 und des Abdeckelements 45 miteinander erfolgt insbesondere durch sogenanntes Bonden bzw. Verschweißen. Jedoch ist hier auch grundsätzlich jede sonstige geeignete Art der Verbindung oder eine Sandwich-Bauweise oder dergleichen möglich.

In einer besonders bevorzugten Ausführungsvariante wird ein nicht dargestelltes Plattenstück, insbesondere ein Silizium-Wafer, verwendet, aus dem eine Vielzahl von Materialstücken 44 für eine Vielzahl von Filtern 42 hergestellt wird. Vor einer Zerlegung in einzelne Materialstücke 44 bzw. Filter 42 werden vorzugsweise zunächst die Strukturen, insbesondere Vertiefungen, ausgehend von einer Flachseite des Plattenstücks für die Vielzahl der Materialstücke 44 hergestellt. Dies erfolgt insbesondere durch das bei der Halbleiterherstellung übliche Herstellen bzw. Ätzen feiner Strukturen, so daß diesbezüglich auf den Stand der Technik für das Ätzen von Silizium oder dergleichen verwiesen werden darf.

Besonders bevorzugt wird das Abdeckelement 45 ebenso wie das Materialstück 44 aus einem Plattenstück hergestellt, das in eine Vielzahl von Abdeckelementen 45 zerlegt bzw. aufgetrennt wird. Für die Herstellung des Materialstücks 44 wird besonders bevorzugt ein Silizium-Wafer als Plattenstück verwendet, wie bereits erläutert. Für das Plattenstück zur Herstellung der Abdeckelemente 45 kann ebenfalls ein Silizium-Wafer oder ein sonstiger Wafer, eine Glasscheibe oder dergleichen eingesetzt werden.

Wenn sowohl für die Herstellung der Materialstücke 44 als auch für die Herstellung der Abdeckelemente 45 jeweils ein Plattenstück eingesetzt wird, erfolgt besonders bevorzugt das Verbinden der Plattenstücke miteinander vor dem Zerlegen in die einzelnen Materialstücke 44 bzw. Abdeckelement 45. Dies erleichtert den Zusammenbau und die Positionierung wesentlich.

Der Filter 42 kann bedarfsweise auch mehrere - also zwei oder mehr - Reihen von Durchlässen 43 hintereinander aufweisen. Die Reihen können beispielsweise parallel zueinander verlaufen. Hierzu können beispielsweise mehrere - also zwei oder mehr - Erhöhungen 52 auf einer Erhebung 51 - insbesondere parallel und/oder beabstandet zueinander verlaufend - vorgesehen sein. Alternativ oder zusätzlich können auch mehrere - insbesondere zwei oder mehr - Erhebungen 51 jeweils zumindest mit einer Erhöhung 52 hintereinander bzw. in Serie - insbesondere parallel und/oder beabstandet zueinander verlaufend - angeordnet sein.

Grundsätzlich kann der Filter 42 auch mit einem sonstigen Filter, wie einem Vorfilter o. dgl., kombiniert - also insbesondere in Serie - geschaltet werden.

Der vorschlagsgemäße Filter 42 kann auch für die Auslaß- oder Düsenbaugruppe 36 - beispielsweise entsprechend der dritten Ausführungsform gemäß Fig. 7 - eingesetzt werden.

Insbesondere ist möglich, den Filter 42 als Baueinheit mit der Austragsdüse 12 bzw. der Auslaß- oder Düsenbaugruppe 36 auszubilden. Dies vereinfacht die Herstellung und Montage wesentlich. Besonders bevorzugt erfolgt dann die Herstellung der Austragsdüse 12 bzw. gegebenenfalls mehrerer Austragsdüsen 12 zusammen mit dem zugeordneten Filter 42, ganz besonders bevorzugt in der oben beschriebenen Weise aus dem Materialstück 44 und dem Abdeckelement 45 oder auf jede sonstige geeignete Art und Weise.

Die vorschlagsgemäße Lösung kann aber nicht nur bei den hier im einzelnen beschriebenen Zerstäubern 1 sondern auch bei sonstigen Zerstäubern oder Inhalatoren, diesbezüglichen Behältern 3 oder dergleichen eingesetzt werden.

Insbesondere kann der vorschlagsgemäße Filter 42 auch für sonstige Zwecke, beispielsweise bei der Reinigung des Fluids 2, generell bei der Abscheidung von Keimen, Bakterien, Zellen oder dergleichen eingesetzt werden. Insbesondere ist der Filter 42 für Kleinmengen oder Kleinstmengen im µl-Bereich geeignet und ausgelegt.

Generell kann der Zerstäuber 1 auch einen sonstigen Filter 42, beispielsweise aus porösem Material, aufweisen, um die Austragsdüse 12 oder die Auslaß- bzw. Düsenbaugruppe 36 oder den Auslaßkanal 37 vor einer möglichen Verstopfung zu schützen.

Einzelne Merkmale, Aspekte oder Eigenschaften der verschiedenen Ausführungsformen können auch beliebig miteinander kombiniert oder bei in sonstigen Zerstäubern, Inhalatoren oder dergleichen eingesetzt werden.

Das Fluid 2 ist vorzugsweise ethanolhaltig und enthält insbesondere Ethanol als Lösungsmittel.

Alternativ oder zusätzlich kann das Fluid 2 EDTA (Ethylendiamintetraessigsäure) bzw. deren Salze als Komplexbildner enthalten.

Vorzugsweise handelt es sich bei dem Fluid 2 um eine Flüssigkeit, wie bereits erwähnt, insbesondere um eine wäßrige, ethanolische oder wäßrig/ethanolische Arzneimittelzubereitung. Es kann sich jedoch auch um eine sonstige Arzneimittelzubereitung, eine Suspension oder dergleichen handeln.

Nachfolgend werden bevorzugte Verbindungen, Bestandteile und/oder Formulierungen des vorzugsweise medizinischen Fluids 2 aufgeführt. Wie bereits erwähnt, kann es sich um wäßrige oder nicht wäßrige Lösungen, Mischungen, ethanolhaltige oder lösungsmittelfreie Formulierungen oder dergleichen handeln. Besonders bevorzugt sind:
Die unten genannten Verbindungen können allein oder in Kombination zur Anwendung in der erfindungsgemäßen Vorrichtung gelangen. In den unten genannten Verbindungen ist **W** einen pharmakologisch, aktiver Wirkstoff und (beispielsweise) ausgewählt aus der Gruppe bestehend aus Betamimetika, Anfach Kombinationen von **W** kombiniert werden und zur Anwendung in der erfindungsgemäßen Vorrichtung gelangen. Beispielhaft genannte Kombinationen von **W** wären:
- **W** stellt ein Betamimetika dar, kombiniert mit einem Anticholinergika, Corticosteroide, PDE4-Inhibitore, EGFR-Hemmern oder LTD4-Antagonisten,
- **W** stellt ein Anticholinergika dar, kombiniert mit einem Betamimetika, Corticosteroiden, PDE4-Inhibitoren, EGFR-Hemmern oder LTD4-Antagonisten,
- **W** stellt ein Corticosteroiden dar, kombiniert mit einem PDE4-Inhibitoren, EGFR-Hemmern oder LTD4-Antagonisten
- **W** stellt ein PDE4-Inhibitoren dar, kombiniert mit einem EGFR-Hemmern oder LTD4-Antagonisten
- **W** stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HO-KU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel **AC-1** 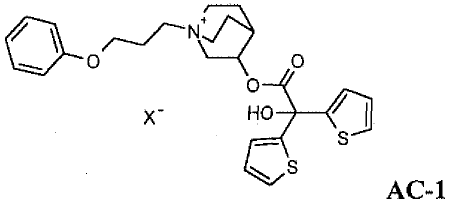 worin X⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 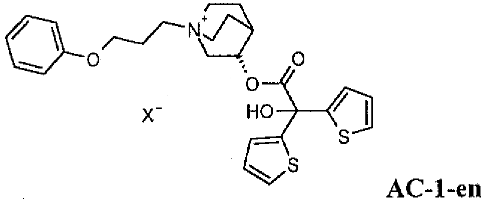 enthalten, worin X⁻ die vorstehend genannten Bedeutungen aufweisen kann. Weiterhin bevorzugte Anticholinergika sind ausgewählt aus den Salzen der Formel **AC-2** 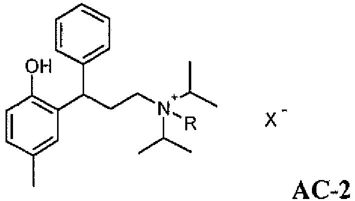 worin R entweder Methyl oder Ethyl bedeuten und worin X⁻ die vorstehend genannte Bedeutungen aufweisen kann. In einer alternativen Ausführungsform kann die Verbindung der Formel **AC-2** auch in Form der freien Base **AC-2-base** vorliegen.

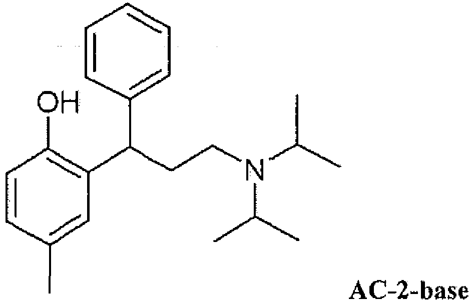

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difluoro-11β-hydroxy-16α-methyl-3-oxo-17α-(2,2,3,3-tertamethylcyclopropylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, CI-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxyphenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methexyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)-propyl)thio)methyl)cyclopropanessigsäure
- [2-[[2-(4-tert-Butyl-2-thiazolyl)-5-benzofuranyl]oxymethyl]phenyl]-essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1 003 478 A1 oder CA 2297174 A1 offenbart.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

**Bezugszeichenliste**

| | | | |
|---|---|---|---|
| 1 | Zerstäuber | 31 | Rastausnehmung |
| 2 | Fluid | 32 | Schutzeinrichtung |
| 3 | Behälter | 33 | Beutel |
| 4 | Beutel | 34 | Aufnahmeraum |
| 5 | Druckerzeuger | 35 | Kontaktabschnitt |
| 6 | Halterung | 36 | Auslaß- bzw. Düsenbaugruppe |
| 7 | Antriebsfeder | 37 | Auslaßkanal |
| 8 | Sperrelement | 38 | Ventil |
| 9 | Förderrohr | 39 | Ventilelement |
| 10 | Rückschlagventil | 40 | Schließfeder |
| 11 | Druckkammer | 41 | Verbindungskanal |
| 12 | Austragsdüse | 42 | Filter |
| 13 | Mundstück | 43 | Durchlaß |
| 14 | Aerosol | 44 | Materialstück |
| 15 | Zuluftöffnung | 45 | Abdeckelement |
| 16 | Gehäuseoberteil | 46 | Einlaß |
| 17 | Innenteil | 47 | Verteilkammer |
| 17a | oberes Teil des Innenteils | 48 | Filterstruktur |
| 17b | unteres Teil des Innenteils | 49 | Sammelkammer |
| 18 | Gehäuseteil (Unterteil) | 50 | Auslaß |
| 19 | Halteelement | 51 | Erhebung |
| 20 | Feder (im Gehäuseunterteil) | 52 | Erhöhung |
| 21 | Behälterboden | 53 | Stützstruktur |
| 22 | Anstechelement | | |
| 23 | Abdeckung | b | Breite (Durchlaß) |
| 24 | Versiegelung | h | Höhe (Durchlaß) |
| 25 | Septum | d | Durchmesser (Durchlaß) |
| 26 | Belüftungsöffnung | l | Länge (Durchlaß) |
| 27 | Sicherungsteil | B | Breite (Erhebung) |
| 28 | Rastarm | | |
| 29 | Rastnase | | |

## Patentansprüche

1. Zerstäuber (1), in Form eines Inhalators zur medizinischen Aerosol-Therapie, zur Zerstäubung eines Fluids (2),
wobei der Zerstäuber (1) einen Behälter (3) mit dem Fluid (2),
eine Austragsdüse (12) zur Zerstäubung des Fluids (2) und
einen Druckerzeuger (5) als Fördereinrichtung zur Förderung und Zerstäubung des Fluid (2) mit einer Halterung (6) für den Behälter (3), einem Förderrohr (9) und einer Antriebsfeder (7) aufweist,
wobei der Druckerzeuger (5) eine Druckkammer (11) zur Erzeugung eines Federdrucks von 5 bis 60 MPa auf das Fluid (2) aufweist,
der Behälter (3) während der Fluidförderung, Druckerzeugung und Zerstäubung hubartig bewegbar ist und
bei der Zerstäubung pro Hub ein Fluid-Volumen von 10 bis 50 µl ausgebracht wird,
**dadurch gekennzeichnet,**
**daß** der Zerstäuber (1) Schutzeinrichtungen (32) zur Verhinderung einer Verkeimung des Fluids (2) aufweist und die Schutzeinrichtungen (32) einen zumindest weitgehend keimdichten Filter (42) und ein sperrbares Ventil (38) zur Sperrung eines Kanalabschnitts zur Führung des Fluids umfassen,
wobei der Filter und das Ventil (38) stromab der Druckkammer (11) angeordnet sind, und
wobei der Filter (42) nur so feine Durchlässe (43) aufweist, daß er zumindest im wesentlichen keimdicht ist, wobei die maximale Breite (b), die maximale Höhe (h) und/oder der maximale Durchmesser (d) der Durchlässe (43) etwa 0,3 µm oder weniger beträgt.

2. Zerstäuber nach Anspruch 1, **dadurch gekennzeichnet, daß** das Ventil (38) unmittelbar benachbart oder stromauf der Austragsdüse (12) angeordnet ist.

3. Zerstäuber nach Anspruch 1, **dadurch gekennzeichnet, daß** die Austragsdüse (12) Teil einer Düsenbaugruppe (36) ist, die einen Auslasskanal (37) mit der sich anschließenden Austragsdüse (12) aufweist, und dass das Ventil (38) den Auslasskanal (37) sperrt.

4. Zerstäuber nach einem der Ansprüche 1 bis 3, **dadurch gekennzeichnet, daß** der Behälter (3) mehrere Dosen des Fluids (2) im Auslieferungszustand enthält.

5. Zerstäuber nach Anspruch 4, **dadurch gekennzeichnet, daß** das Fluid (2) konservierungsmittelfrei ist.

6. Zerstäuber nach Anspruch 4 oder 5, **dadurch gekennzeichnet, daß** der Behälter (3) im Auslieferungszustand bereits in den Zerstäuber (1) eingesetzt, aber insbesondere noch keimdicht verschlossen ist.

7. Zerstäuber nach einem der Ansprüche 4 bis 6 **dadurch gekennzeichnet, daß** bereits im Auslieferungszustand des Zerstäubers (1) der verschlossene Behälter (3) im Zerstäuber (1) angeordnet und der Zerstäuber (1) derart ausgebildet ist, daß der Behälter (3) vor oder bei der erstmaligen Benutzung des Zerstäubers (1) innerhalb des Zerstäubers (1) geöffnet wird.

8. Zerstäuber nach einem der Ansprüche 4 bis 7 **dadurch gekennzeichnet, daß** der Zerstäuber (1) derart ausgebildet ist, daß der Behälter (3) nicht auswechselbar, insbesondere nicht entnehmbar, ist.

9. Zerstäuber nach einem der voranstehenden Ansprüche, **dadurch gekennzeichnet, daß** das Ventil (38) selbsttätig, vorzugsweise durch Federkraft schließt.

10. Zerstäuber nach Anspruch 9, **dadurch gekennzeichnet, daß** das Ventil (38) durch anstehenden Fluiddruck öffenbar ist.

11. Zerstäuber nach einem der vorangegangenen Ansprüche, **dadurch gekennzeichnet, daß** der Filter (42) Durchlässe (43) aufweist, die durch Ätzen, Prägen und/oder Laserbearbeitung hergestellt sind.

12. Zerstäuber nach einem der vorangegangenen Ansprüche, **dadurch gekennzeichnet, daß** der Filter (42) aus Sintermaterial, Keramik, Glas und/oder Silizium hergestellt ist.

13. Zerstäuber nach einem der vorangegangenen Ansprüche, **dadurch gekennzeichnet, daß** der Filter (42) eine Baueinheit mit der Austragsdüse (12) bildet.

## Claims

1. Atomiser (1), in the form of an inhaler for medical aerosol therapy, for atomising a fluid (2),
the atomiser (1) comprising a container (3) holding the fluid (2),
a discharge nozzle (12) for atomising the fluid (2), and
a pressure generator (5) as a conveying means for conveying and atomising the fluid (2) having a holder (6) for the container (3), a conveyor tube (9) and a drive spring (7),
the pressure generator (5) having a pressure chamber (11) for generating a spring pressure of from 5 to 60 MPa on the fluid (2),
the container (3) being movable in a stroke-like manner during the fluid conveyance, pressure generation and atomisation and,
during atomisation, a fluid volume of from 10 to 50 µl per stroke being released,
**characterised in that**
the atomiser (1) has protection devices (32) for preventing bacterial contamination of the fluid (2) and the protection devices (32) comprise an at least largely germ-proof filter (42) and a lockable valve (38) for blocking a channel portion for guiding the fluid,
the filter and the valve (38) being arranged downstream of the pressure chamber (11), and
wherein the filter (42) has such fine openings (43), that it is at least substantially germ-prove, wherein the maximum width (b), the maximum height (h) and/or the maximum diameter (d) of the openings (43) is about 0.3 nm or less.

2. Atomiser according to claim 1, **characterised in that** the valve (38) is arranged directly adjacent to or upstream of the discharge nozzle (12).

3. Atomiser according to claim 1, **characterised in that** the discharge nozzle (12) is part of a nozzle assembly (36), which has an outlet channel (37) adjoining the discharge nozzle (12), and **in that** the valve (38) blocks the outlet channel (37).

4. Atomiser according to any of claims 1 to 3, **characterised in that** the container (3) contains a plurality of doses of the fluid (2) in the delivery state.

5. Atomiser according to claim 4, **characterised in that** the fluid (2) is free of preservatives.

6. Atomiser according to either claim 4 or claim 5, **characterised in that** the container (3) is already installed in the atomiser (1) in the delivery state, but in particular is still sealed so as to be germ-proof.

7. Atomiser according to any of claims 4 to 6, **characterised in that** the sealed container (3) is already arranged in the atomiser (1) in the delivery state of the atomiser (1) and the atomiser (1) is designed such that the container (3) is opened within the atomiser (1) before or during the first use of the atomiser (1).

8. Atomiser according to any of claims 4 to 7, **characterised in that** the atomiser (1) is designed such that the container (3) is not replaceable, and in particular is not removable.

9. Atomiser according to any of the preceding claims, **characterised in that** the valve (38) closes automatically, preferably by spring force.

10. Atomiser according to claim 9, **characterised in that** the valve (38) can be opened by means of pending fluid pressure.

11. Atomiser according to any of the preceding claims, **characterised in that** the filter (42) has openings (43) which are produced by etching, embossing and/or laser machining.

12. Atomiser according to any of the preceding claims, **characterised in that** the filter (42) is made from sintered material, ceramic material, glass and/or silicon.

13. Atomiser according to any of the preceding claims, **characterised in that** the filter (42) forms a structural unit together with the discharge nozzle (12).

## Revendications

1. Pulvérisateur (1) se présentant sous la forme d'un inhalateur pour la thérapie médicale par aérosol, servant à pulvériser un fluide (2),
dans lequel le pulvérisateur (1) présente un contenant (3) avec le fluide (2),
une buse de distribution (12) servant à pulvériser le fluide (2) et
un générateur de pression (5) en tant que dispositif de refoulement servant à refouler et à pulvériser le fluide (2) avec une fixation (6) pour le contenant (3), un tube de refoulement (9) et un ressort d'entraînement (7),
dans lequel le générateur de pression (5) présente une chambre de pression (11) servant à générer une pression de ressort de 5 à 60 MPa sur le fluide (2),
le contenant (3) peut être déplacé selon une course pendant le refoulement de fluide, la génération de pression et la pulvérisation, et
un volume de fluide de 10 à 50 µl est distribué par course lors de la pulvérisation,
**caractérisé en ce**
**que** le pulvérisateur (1) présente des dispositifs de protection (32) servant à empêcher une contamination du fluide (2) et les dispositifs de protection (32) comprennent un filtre (42) au moins largement étanche aux germes et une soupape (38) pouvant être bloquée servant à bloquer un tronçon de canal servant à acheminer le fluide,
dans lequel le filtre et la soupape (38) sont disposés en aval de la chambre de pression (11), et
dans lequel le filtre (42) a des ouvertures (43) si fines qu'il est au moins substantiellement imperméable aux germes, la largeur maximale (b), la hauteur maximale (h) et/ou le diamètre maximal (d) des ouvertures (43) étant d'environ 0,3 nm ou moins.

2. Pulvérisateur selon la revendication 1, **caractérisé en ce que** la soupape (38) est disposée directement de manière adjacente ou en amont de la buse de distribution (12).

3. Pulvérisateur selon la revendication 1, **caractérisé en ce que** la buse de distribution (12) fait partie d'un module de buses (36), qui présente un canal d'évacuation (37) avec la buse de distribution (12) s'y raccordant, et que la soupape (38) bloque le canal d'évacuation (37).

4. Pulvérisateur selon l'une quelconque des revendications 1 à 3, **caractérisé en ce que** le contenant (3) contient plusieurs doses du fluide (2) dans l'état de délivrance.

5. Pulvérisateur selon la revendication 4, **caractérisé en ce que** le fluide (2) est exempt d'agent de conservation.

6. Pulvérisateur selon la revendication 4 ou 5, **caractérisé en ce que** le contenant (3) est déjà inséré dans le pulvérisateur (1) dans l'état de délivrance, mais en particulier est encore fermé de manière étanche aux germes.

7. Pulvérisateur selon l'une quelconque des revendications 4 à 6, **caractérisé en ce que** déjà dans l'état de délivrance du pulvérisateur (1), le contenant (3) fermé est disposé dans le pulvérisateur (1), et le pulvérisateur (1) est réalisé de telle manière que le contenant (3) est ouvert avant ou lors de l'utilisation pour la première fois du pulvérisateur (1) à l'intérieur du pulvérisateur (1).

8. Pulvérisateur selon l'une quelconque des revendications 4 à 7, **caractérisé en ce que** le pulvérisateur (1) est réalisé de telle manière que le contenant (3) n'est pas remplaçable, en particulier ne peut être retiré.

9. Pulvérisateur selon l'une quelconque des revendications précédentes, **caractérisé en ce que** la soupape (38) se ferme de manière autonome, de préférence par la force de ressort.

10. Pulvérisateur selon la revendication 9, **caractérisé en ce que** la soupape (38) peut être ouverte par une pression de fluide présente.

11. Pulvérisateur selon l'une quelconque des revendications précédentes, **caractérisé en ce que** le filtre (42) présente des passages (43), qui sont fabriqués par gravure, estampage et/ou usinage laser.

12. Pulvérisateur selon l'une quelconque des revendications précédentes, **caractérisé en ce que** le filtre (42) est fabriqué à partir d'un matériau de frittage, de céramique, de verre et/ou de silicium.

13. Pulvérisateur selon l'une quelconque des revendications précédentes, **caractérisé en ce que** le filtre (42) forme une unité modulaire avec la buse de distribution (12).
